# EUROPEAN PATENT SPECIFICATION

(11) **EP 1 161 226 B1**
(45) Date of publication and mention of the grant of the patent: **26.05.2004**
(21) Application number: 00908713.1
(22) Date of filing: 16.02.2000
(51) Int. Cl.: A61K 9/127, A61K 31/665, A61K 31/675, A61K 31/685, C07D 259/00, C07D 487/22, C07F 9/02, A61K 47/48

(54) **PHOSPHOCHOLINE LINKED PRODRUG DERIVATIVES**
AN PHOSPHOCHOLIN GEBUNDENE PRODRUG-DERIVATE
DERIVES DE PROMEDICAMENT LIES A LA PHOSPHOCHOLINE

(30) Priority: 18.02.1999 US 120483 P
(43) Date of publication of application: 12.12.2001
(73) Proprietor: SuperGen, Inc., Dublin, CA 94568 (US)
(72) Inventor: MORIMOTO, Bruce, H., Redwood City, CA 94061 (US); BARKER, Peter, L., El Granada, CA 94018 (US)
(74) Representative: Marchant, James Ian
(86) International application number: PCT/US2000/004140
(87) International publication number: WO 2000/048572

(56) References cited:
- WO-A-96/16680
- WO-A1-98/11906
- US-A- 5 534 499
- US-A- 5 830 432

## Description

### FIELD OF THE INVENTION

The present invention is directed to a novel class of phosphocholine-linked derivatives which not only increase water solubility, but also function as true prodrugs, allow phosphocholines or phosphocholine congeners to be attached to a variety of functional groups on the therapeutic agent, and have the potential on being able to control the rate of release of the pharmaceutical agent.

### BACKGROUND OF THE INVENTION

Conventional means for delivering pharmaceutical and therapeutic agents to mammals often are severely limited by chemical and physical properties of the agent, such as aqueous solubility. For example, oral delivery of many biologically-active agents would be the route of choice if not for poor bioavailability due to the limited dissolution of the active agent and subsequent absorption.

Water insoluble therapeutic agents are particularly difficult to administer parenterally. Formulations often require inclusion of a variety of emulsifiers, such as CREMOPHOR® EL. But CREMOPHOR® , which is poly(oxyethylene)-40-castor oil, can result in hypotension, dyspnea, angioedema, or generalized urticaria. These hypersensitive reactions can lead to life-threatening conditions, and it is recommended that all patients be premedicated with corticosteroids, diphenhydramine, and H2 antagonists to avoid severe hypersensitivity.

Another emulsifier is administered in Propofol, an anesthetic. This emulsion contains soy bean oil, glycerol, and egg phosphatide that create a microbial contamination problem with the current formulation of propofol, which can result in life-threatening illness or death from fever, infection or sepsis. This is especially problematic for post-operative or intensive care unit (ICU) patients. Although U.S. Patent 5,714,120 discloses a method to minimize microbial contamination by the addition of a preservative, this formulation is not an antimocrobial preserved product by USP standards and extrinsic contamination remains problematic.

U.S. Patent 5,534,499 provides novel compounds which are capable of being formulated in liposomes or micelies. The compounds of the invention are therapeutic agents which are covalently attached to a fatty acid chain of a phospholipid, glyceride, ceramide or 1,2-diacyloxypropane-3-amine. The linkage between the therapeutic agent and the lipid is selected such that the therapeutic agent can be cleaved from the lipid in vivo.

There is thus a need in the art for methods and compositions to enable potential therapeutic agents to be rendered soluble thereby circumventing the need for emulsifiers and providing for safer and more efficacious therapeutic agents.

### SUMMARY OF THE INVENTION

In one aspect, the present invention provides a method for enabling potential therapeutic agents to be rendered soluble comprising the steps of inserting one or more linker moieties having one or more primary alcohol group between a phosphocholine or a phosphocholine congener to the therapeutic agents having one or more compatible group.

In another aspect, the present invention provides a method for increasing the bioavailability of a pharmaceutical agent on administration to a mammal, comprising the steps of derivatizing the agent with one or more linker moieties, producing an intermediate, recovering and coupling the intermediate with phosphocholine or a phosphocholine-congener to the linkers and producing a final derivative, wherein the agent in derivative form is significantly more soluble in aqueous media than the agent in non-derivatized form.

In yet another aspect, the present invention provides a composition of matter comprising an isolated phosphocholine linked via a linker or propofol, a sedative or anesthetic agent.

In yet another aspect, the present invention provides a pharmaceutical formulation for treating a mammal suffering from cancer comprising an isolated phosphocholine linked via a linker to paclitaxel and a physiologically acceptable vehicle, carrier, binder, preservative, stabilizer, flavor, etc., as called for by accepted pharmaceutical practice, wherein the linker is one or more of the groups selected from the group consisting of (i) substituted or unsubstituted alkyl, (ii) substituted or unsubstituted alkenyl, (iii) substituted or unsubstituted alkanoyl, (iv) substituted or unsubstituted alkenoyl wherein the double bond is cis, and (v) (ortho or para) carbonyl-substituted aryl; and wherein the substituent is each an independent group of linked together thereby forming a ring.

The aqueous solubilities of the compounds described herein are evaluated by several methods known in the art, such as preparing a saturated solution of the compound in water, removing a known volume of the solution, and quantitating the amount of the compound in that solution using standard analytical techniques, like HPLC or LC-MS.

These and other aspects of the present invention will be apparent to those of ordinary skill in the art in light of the present description, claims and drawings.

### DETAILED DESCRIPTION OF THE INVENTION

The invention in its broad aspects relates to phosphocholine or phosphocholine congeners, attached via a linker, to a therapeutic agent.

Phosphocholine derivatives of therapeutic agents containing a primary alcohol or a phenol are readily cleaved by phosphatases and mammalian esterases. The preparation of phosphocholine derivatives of biologically active agents has been reported (e.g., U.S. Patent No. 5,703,063). However, if the phosphocholine is attached to a secondary or sterically hindered alcohol, hydrolysis or removal of the phosphocholine does not occur rapidly or to a large extent.

The present invention advantageously provides insertion of a linker between the phosphocholine and the secondary alcohol of the therapeutic agent wherein the phosphocholine is bound to the linker via a primary alcohol or phenol functional group inherent in the linker. This formulation facilitates enzymatic cleavage of the phosphocholine linker bond and liberates the primary alcohol or phenol of the linker. The linker then spontaneously eliminates to liberate the therapeutic agent and an inert molecule arising from the decomposed linker.

Poor water solubility of biologically active agents is, in many cases, the reason for poor bioavailability of the compounds. The compounds described herein display no less than 5 to 10-fold increased biological activity and/or aqueous solubility as compared to the non-derivatized therapeutic agents when administered by the same route. Preferably, the compounds display increased biological activity and/or aqueous solubility in the range from one hundred fold to one hundred thousand fold relative to the non-derivatized therapeutic agents. Thus, the compounds described herein are useful for the enhanced bioavailability of otherwise water-insoluble compounds.

The phosphocholine congeners include, but are not limited to, *O*-phosphoserine; *O*-phosphothreonine; *O*-phosphotyrosine and their mono- and di-*N*-methyl derivatives; *O*-phosphoethanolamine and their mono- and di-*N*-methyl derivatives.

Although the compounds of this invention can include phosphocholine or phosphocholine congeners, they will be described below as compounds having phosphocholine of general **FORMULA I:** 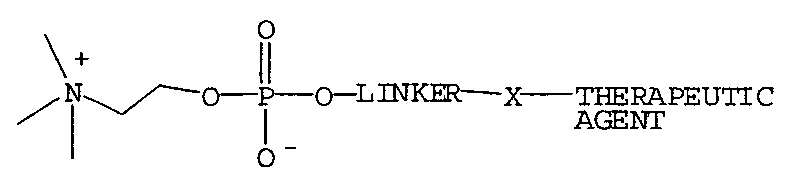 wherein the LINKER is one or more of the groups selected from the group consisting of (i) substituted or unsubstituted alkyl, (ii) substituted or unsubstituted alkenyl, (iii) substituted or unsubstituted alkanoyl, (iv) substituted or unsubstituted alkenoyl wherein the double bond is *cis,* and (v) (*ortho* or *para*) carbonyl-substituted aryl; and
wherein the subtituent is each an independent group or linked together thereby forming a ring; and
wherein X is one or more substituted or unsubstituted group containing one or more O, N, or S atom and
wherein the substituent is each an independent group or linked together thereby forming a ring; and
wherein the therapeutic agent is selected from the group consisting of alcohol-containing water-insoluble steroids and another alcohol containing compounds.

The (*ortho* or *para*) carbonyl-substituted aryl of the LINKER is selected from the group consisting of *ortho*-CR₁R₂-substituted aryl-CO, substituted aryl-*ortho*-CR₃R₄-CO, substituted aryl-*ortho*- CR₃R₄-CR₅R₆-CO, substituted aryl-*ortho*-CR₃=CR₄-CO wherein the double bond is *cis*, *ortho*-CR₁R₂-substituted aryl-CR₅R₆-CO, and substituted aryl-(*ortho* or *para*)-CO.

The aryl substituents may optionally be selected to accelerate or decelerate the rate of enzymatic cleavage of a phenolic phosphocholine. Examples of substituents accelerating the rate of enzymatic cleavage would be nitro, alkyl or aryl sulfonyl, alkyl or aryl keto, alkyl or aryl oxycarbonyl in the ortho and/or para positions relative to the phenolic phosphocholine. Examples of substituents decelerating the rate of enzymatic cleavage of a phenolic phosphocholine would be alkyl, alkoxy, alkylthio in the ortho and/or para positions relative to the phenolic phosphocholine.

The aryl is selected from the group consisting of benzene, naphthalene, pyridine, pyrrole, thiophene, furan, imidazole, thiazole, oxazole, pyrimidine, indole, benzimidazole, benzthiazole, benzofuran, benzothiophene and quinoline, each bearing one or more of the group consisting of hydrogen, C₁₋₈-alkyl, C₁₋₈-alkoxy, F, Cl, Br, C₁₋₈-alkoxycarbonyl, amino, substituted amino, nitro, C₁₋₈-alkylthio, C₁₋₈-alkyl sulfoxido, and C₁₋₈-alkylsulfono.

In one embodiment, the present invention is a compound having a general formula I wherein (i) said alkyl has the formula CR₁R₂, (ii) said alkenyl has the formula CR₁=CR₃-CR₄, (iii) said alkanoyl has the formula CR₁R₂-CR₃R₄-CR₅R₆-CO, (iv) said alkenoyl has the formula CR₁R₂-CR₃=CR₄-CO and wherein the double bond is *cis,* and (v) said substituted aryl has the formula aryl-CR₁R₂; and
wherein R₁, R₂, R₃, R₄, R₅, and R₆ are the same or different and are selected from the group consisting of
(i) hydrogen;
(ii) linear, branched, and unsaturated C₁₋₁₂-alkyl;
(iii) substituted C₁₋₈-alkyl, wherein the substituent is selected from the group consisting of Y1-Y24, wherein
   Y1 is hydroxy,
   Y2 is C₁₋₈-alkoxy,
   Y3 is carbo-C₁₋₈-alkoxy,
   Y4 is C₁₋₈-alkylamino,
   Y5 is di-C₁₋₈-alkylamino,
   Y6 is C₆₋₁₂-arylamino,
   Y7 is C₆₋₁₂-aryloxy,
   Y8 is amino,
   Y9 is amino-C₂-C₈-alkoxy,
   Y10 is C₁₋₈-alkylthio,
   Y11 is C₆₋₁₂-arylthio,
   Y12 is acetamido,
   Y13 is mercapto,
   Y14 is benzamido,
   Y15 is carboxamido,
   Y16 is phthalimido,
   Y17 is guanidino,
   Y18 is ureido,
   Y19 is isothioureido,
   Y20 is carboxy,
   Y21 is (C₆₋₁₂) aryl - (C₁₋₈) alkyl,
   Y22 is (C₆₋₁₂)aryl- (C₂₋₈) alkenyl,
   Y23 is aromatic heteracyclo (C₁₋₈) alkyl,
   and Y24 is aromatic heterocyclo-(C₂₋₈)-alkenyl wherein the heterocyclic group of Y23 and Y24 have 5-10 ring atoms and have up to two O, N, or S heteroatoms;
and(iv) substituted Y21 or substituted Y23 wherein the substituent is selected from the group consisting of Y1, Y2, Y4, Y5, Y7, Y8, Y12, Y14, Y17-Y20, and Y25-Y29 wherein
   Y25 is halogen,
   Y26 is C₁₋₈-alkyl,
   Y27 is amino-C₁₋₈-alkyl,
   Y28 is C₆₋₁₂-aroyl, and
   Y29 is C₁₋₈-alkanoyl.

Unless specified otherwise:(i) alkyl, alkenyl and alkynyl denote straight and branched hydrocarbon chains having single, double and triple bonds, respectively; (ii) C₆₋₁₂-aryl groups denote unsubstituted aromatic ring or rings such as, for example, phenyl or naphthyl; (iii) hetero denotes the heteroatoms O, N, or S; (iv) aromatic heterocyclic have five to ten ring atoms and contain up to four heteroatoms; (v) halogen or halo denote F, Cl, Br, or I atoms; and (vi) alkoxy denotes an alkyl group attached to O.

Examples of C₁₋₈-alkyl or C₂₋₈ alkenyl groups include methyl, ethyl, propyl, isopropyl, butyl, t-butyl, sec-butyl, pentyl, isopentyl, hexyl, vinyl, allyl, butenyl and the like; aromatic heterocyclic group is selected from the group consisting of pyridyl, thienyl, furyl, indoyl, benzthienyl, imidazoyl, thiazolyl, quinolyl and isoquinoyl.

The compounds containing the R-groups described herein can be purchased from numerous commercial sources such as Sigma Chemical Company (St. Louis, MO), Aldrich Chemical Company (Milwaukee, WI), Acros Organic Chemicals (Pittsburgh, PA), or Fluka Chemical Corporation (Milwaukee, WI). All other compounds not directly available from commercial sources can be prepared from commercially available starting materials by anyone skilled in the art of synthetic organic chemistry.

The preferred linkers are the compounds wherein R₁ is hydrogen, and R₂, R₃, R₄, R₅ and R₆ are the same or different and are selected from the group as defined above.

The most preferred linkers are compounds of the above formula wherein R₁ and R₂ are hydrogen and R₃, R₄, R₅ and R₆ are the same or different and are selected from the group as defined above.

More than one linker per therapeutic agent molecule can be present when more than one appropriate functional group (X) exists. In such case, the order of removal of multiple phosphocholines on a single therapeutic agent would depend on a number of factors: (i) steric effects, (ii) nature of linker, (iii) nature of X. Steric effects influencing the order of removal of multiple phosphocholines would be determined by the immediate steric environment of the specific phosphocholine-linked therapeutic agent. Sterically crowded phosphocholine-linked therapeutic agents would be predicted to be enzymatically cleaved more slowly than non-sterically crowded phosphocholine-linked therapeutic agents. Substituents on the linker may drive the elimination of the linker by sterically favoring a geometric form of the intermediate linker-therapeutic agent which self-eliminates more rapidly. Variables in the nature of the linker include inherent differences in the kinetics of the decomposition of the various linkers, and include the nature of substituents of substituted phenyl based linker as described (above/below). Variables in the nature of X include electronic effects of X as a leaving group. Generally, the more electronically deficient X is a better leaving group and hence is eliminated more rapidly and regenerates the therapeutic agent faster than an electronically rich X.

X is selected the group containing one or more O, N, or S atom selected from the group consisting of O, (O) CO, NR₈, NR₈ CO, NR₈ CO NR₉, NR₈ (SO₂), NR₈ CS, NR₈ CS NR₉, ONR₈, ONR₈CO, NR₈(O), NR₈(O)CO, nitrogen heterocycles, amide and urea internal in the therapeutic agent.

R₈ and R₉ are the same or different and are selected from the group consisting of
(i) hydrogen;
(ii) linear, branched, and unsaturated C₁₋₁₂-alkyl;
(iii) substituted C₁₋₈-alkyl, wherein the substituent is selected from the group consisting of Y1-Y13 and Y15-Y25;
(iv) substituted Y21 or substituted Y23 wherein the substituent is selected from the group consisting of Y1, Y2, Y4, Y5, Y7, Y8, Y12, Y14, Y17-Y20, and Y25-Y29.

There may be more than one X in the therapeutic agent and, hence, more than one phosphocholine linked to the therapeutic agent.

R₈ and R₉ may be linked together thereby forming
(i) a ring of three to six carbon atoms, or
(ii) a ring of two to five carbon atoms and one O, or S heteroatom, or substituted heteroatom NR₇; wherein R₇ is selected from the group consisting of Y21, Y26, and Y28-Y31.

R₈ and / or R₉ may be connected to the therapeutic agent molecule thereby forming alkylene bridge of from one to five carbon atoms and one or two O, S or NR₇ heteroatoms; wherein R₇ is selected from the group consisting of Y21, Y26, Y28-Y31, and the pharmaceutically acceptable salts thereof.

### Examples of therapeutic agents which benefit from a phosphocholine linker:

Numerous biologically active compounds suffer from low water solubility and poor bioavailability. One family of such compounds are steroids, which are in general, poorly bioavailable. The steroids include testosterone, cardiotonic steroids, and other steroids with biological activity.

Testosterone is prescribed therapeutically for men with low levels of endogenous testosterone. Delivery is problematic, however, and necessitates the use of, for example, a testosterone impregnated patch which must be applied directly to the shaven scrotum. A water soluble phosphocholine-linked prodrugs of testosterone could therefore be useful in circumventing delivery of the therapeutic agent.

Cardiotonic steroids, such as digitoxigenin, digoxigenin and ouabugenin are currently used therapeutically. However, their low levels of oral availability makes dosing difficult and the potential for an overdose an important consideration for the attending physician. Phosphocholine linked steroids have the potential to be delivered intravenously, nasally, perorally, intratracheally, administered by patch, etc.

Other steroids with biological activity are candidates for derivatization with phosphocholine linkers. Dehydroepiandrosterone (DHEA), etiocholanolone, pregnenolone, estradiol, estrone, dexamethasone and hydrocortisone are a few examples of steroids which could benefit by deriavatization with a phosphocholine linker.

Anti-neoplastic agents, for example, paclitaxel and other taxanes, etoposide, vincristine, vinblastine, and topoisomerase I inhibitors like camptothecin, irinotecan (Pharmacia & Upjohn, Kalamazoo, MI), topotecan (SmithKline Beecham, Philadelphia, PA), CPT11 (Bristol-Myers Squibb, Princeton, NJ); antiviral agents, including nucleoside analogs and protease inhibitors, such as nelfinavir (Agouron, LaJolla, CA), saquinavir (Roche, Nutley, NJ), crixivan (Merck, West Point, PA), ritonavir (Abbott, N. Chicago, IL); antibiotics, particularly mitomycin, bleomycin, daunorubicin, doxorubicin, actinomycin, and amphotericin; anesthetics, such as propofol and barbituates, for use in general anesthesia or sedation; analgesics, such as morphine, codeine, and Ziconotide (Neurex, Menlo Park, CA); therapeutic peptides or peptidomimetics, composed of D-amino acids, L-amino acids, or amino acid analogs, acting as enzyme inhibitors, receptor ligands, or disruptors of protein-protein interactions, such as cyclosporin A; therapeutic polypeptides. or proteins, such as leptin, growth hormone, calicitonin, vasopressin, renin, prolactin, thyroid and parathyroid hormones, corticotropin, corticotropin-releasing factor, follicle stimulating hormone, luteinizing hormone, gonadotropin, atrial peptides, isolated from natural sources or produced by recombinant DNA technology; nucleic acids, such as anti-sense oligonucleotides or nucleic acids for gene therapy, composed of ribo- or deoxyribonucleotides or nucleotide analogs. Unless otherwise noted, the compounds described herein can be purchased from numerous commercial sources, such as Sigma Chemical Company (St. Louis, MO, Calbiochem-Novabiochem (San Diego, CA), Research Biochemicals Inc. (Natick, MA), or Alexis Corp. (San Diego, CA).

Particularly preferred therapeutic agents for use in the present invention are Propofol and related anesthetic/sedative compounds. These compounds can be conjugated to phosphocholine or phosphocholine congeners via one or more linker pursuant to the present invention and used as anesthetic compounds. It is expected that these derivatized agents will be more effective due to their increased aqueous solubility.

### Compounds of Formula I in pharmaceutical compositions

The derivatized prodrugs of the present invention can be incorporated into pharmaceutical formulations to be used to treat mammals. Pharmaceutical formulations comprising the phosphocholine linked prodrugs derivatives of the present invention as one or more of the active ingredients, would in addition optionally comprise pharmaceutically-acceptable carriers, diluents, fillers, salts and other materials well-known in the art depending upon the dosage form utilized. The compounds of this invention may be utilized in compositions such as tablets, capsules or elixirs for oral administration; suppositories for rectal administration; sterile solutions or suspensions for injectable administration; sterile solutions for ocular or internasal administration, and the like.

Animals in need of treatment using compounds of this invention can be administered dosages that will provide optimal efficacy. The dose and method of administration will vary from animal to animal and be dependent on such factors as weight, diet, concurrent medication and other factors which those skilled in the medical arts will recognize.

Typical formulation of compounds of Formula I as pharmaceutical compositions are discussed below. It will be appreciated that the unit content of active ingredient or ingredients contained in an individual dose or dosage form need not in itself constitute an effective amount for the various usages of the phosphocholine linked prodrugs derivatives of the present invention since the necessary effective amount can be reached by administration of a plurality of such dosage forms.

About 0.5 to 100 mg of a compound or mixture of compounds, as the zwitterionic phosphocholine or as a pharmaceutically acceptable salt, is compounded with a physiologically acceptable vehicle, carrier, binder, preservative, stabilizer, flavor, etc., as called for by accepted pharmaceutical practice. The amount of active ingredients in these compositions is such that a suitable dosage in the range indicated is obtained.

Typical adjuvants which may be incorporated into tablets, capsules and the like are a binder such as acacia, corn starch or gelatin; an excipient such as microcrystalline cellulose; a disintegrating agent like corn starch or alginic acid; a lubricant such as magnesium stearate; a sweetening agent such as peppermint, wintergreen or cherry. When the dosage form is in a capsule, in addition to the above materials it may also contain a liquid carrier such as a fatty oil.

Other materials of various types may be used as coatings or as modifiers of the physical form of the dosage unit. A syrup or elixir may contain the active compound, a sweetener such as sucrose, preservatives such as propyl paraben, a coloring agent.and a flavoring agent such as cherry. Sterile compositions for injection can be formulated according to conventional pharmaceutical practice. For example, dissolution or suspension of the active compound in a vehicle such as water or naturally occurring vegetable oil like sesame, peanut, or cottonseed oil or a synthetic fatty vehicle like ethyl oleate or the like may be desired.

Buffers, preservatives, antioxidants and the like can be incorporated according to the acceptable pharmaceutical practice.

The products of Formula I can be made by using the following general synthetic scheme. The definitions of the substituent groups are the same as for Formula I except where noted. The following examples of reagents are intended to further illustrate the present invention without limiting it thereof.

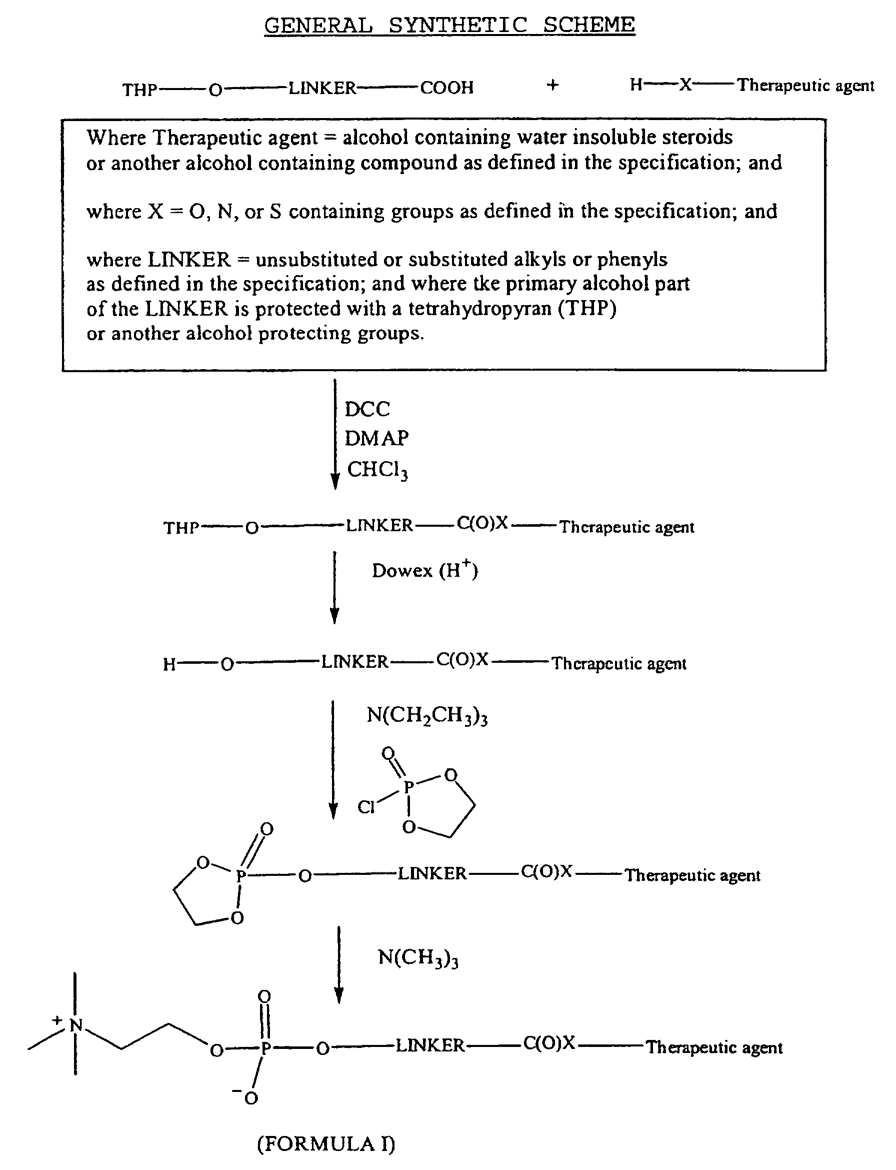

The preferred products of Formula I can also be made by using the method depicted below. The definitions of the substituent groups are the same as for Formula I except where noted.

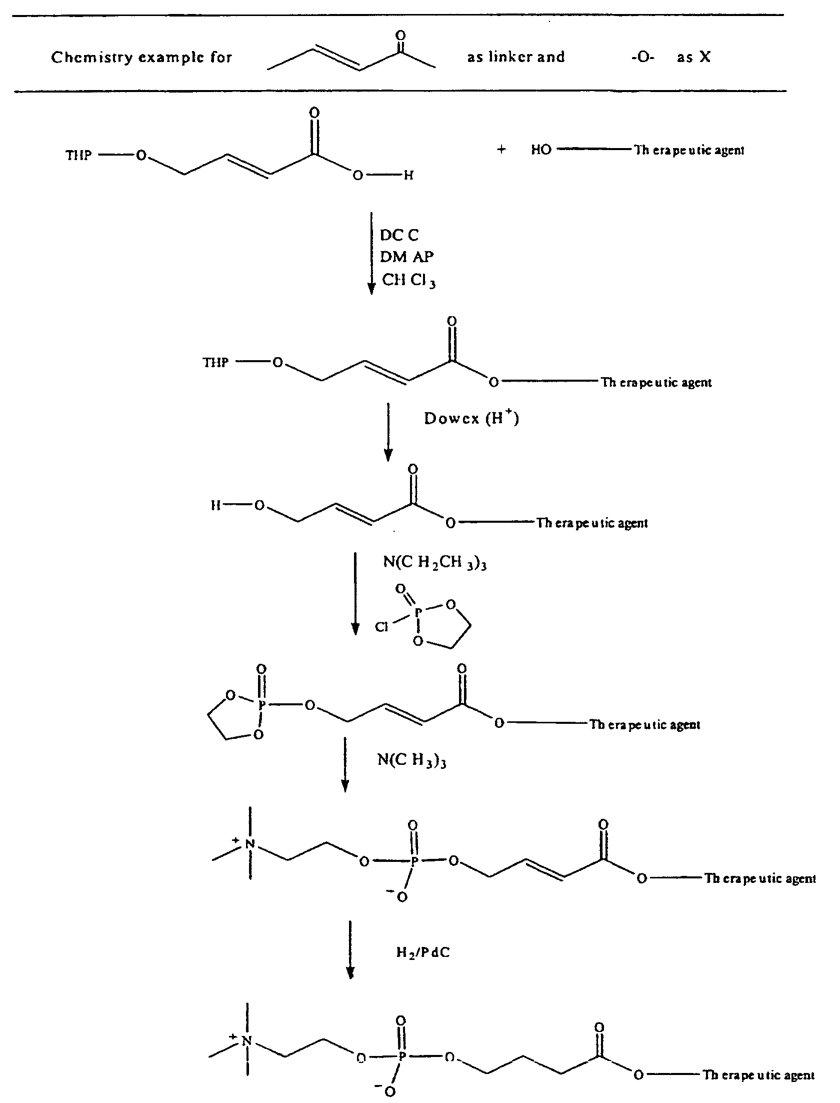

The following examples of compounds of Formula I by using Propofol as a therapeutic agent are illustrated in Example 1 below in Methods A or B. The biological activity of phosphocholine linked prodrugs derivatives of the present invention are illustrated in the Example 2. Both examples are intended to further illustrate the present invention without limiting it thereof. The definitions of the substituent groups are the same as for Formula I except where noted.

### Example 1

### Method A

### Preparation of Phosphocholine-linked Propofol (sedative/anesthetic){2', 6'-Diisopropylphenyl 4-(2-trimethyl ammonium ethyloxy) phosphonobutyrate}.

Ethyl 4-hydroxycrotonate (*trans*) (Kende, Org. Syn. Col. Vol. VII, p221) was treated with 2,3-dihydropyran and catalytic toluenesulfonic acid, according to Bernady (J. Org. Chem. 44, 1438, 1979) to yield ethyl 4-[2-tetrahydro pyranyl]oxycrotonate (*trans*). This compound was further treated with 0.1M LiOH in tetrahydrofuran, to yield the free acid (4-[2-tetrahydropyranyl] oxycrotonic acid), after acidification and work-up. This carboxylic acid was then coupled, via an ester bond, to 2,6-diisopropylphenol, utilizing *N,N*-dicyclohexyl-carbodiimide. After chromatographic purification on silica gel, the ester was then treated, in methanol, with a catalytic amount of Dowex 50W ion exchange resin to affect the removal of the tetrahydropyranyl protecting group. The resulting alcohol was treated with 2-chloro-2-oxo-1,3,2-dioxaphospholane in the presence of triethylamine in chloroform. Upon completion of this reaction, the isolated phosphate intermediate was dissolved in acetonitrile, charged with trimethylamine, and heated @ 80°c for 72 hours. After removal of trimethylamine, the solvent was removed in vacuo, the residue was partitioned between ethyl acetate and water. Freezing and lyophylization of the aqueous phase yielded the 4-*O*-phosphocholine. This compound was then hydrogenated in water to yield the title compound. LC/MS, NMR, and combustion data are available.

### Method B

### Preparation of Phosphocholine-linked Propofol (Sedative, anesthetic) {2', 6'-Diisopropylphenyl 3-[ortho-(O-trimethylammoniumethyl phosphonooxy)]propionate}

To a solution of 3g of 2-hydroxycinnamic acid (trans) in 60mL of dry chloroform was added 7.6mL (eq) of triethylamine. This solution was cooled in an ice bath and 5.7g (2.2eq) of 2- chloro-1,3,5-dioxaphospholane-2-oxide was added dropwise at 0°C. The reaction was allowed to stir at room temperature for thirty minutes. A solution of 2,6 diisopropylphenol (Propofol) in 20mL of chloroform was added and the reaction was stirred at room temperature for 16hr. The reaction was then washed three times with water, and then dried (MgSO₄). Filtration and evaporation of the solvent yielded 10.5g of crude 2',6'-diisopropylphenyl 3-[ortho-(*O*-ethylene phosphonooxy)]propionate. This intermediate was treated with excess trimethylamine in acetonitrile in a pressure vessel at 80°C for 72hr. Removal of the excess trimethylamine and evaporation of the acetonitrile yielded the crude phosphocholine derivative of 2',6'- diisopropyl phenyl 2-hydroxycinnamate. This intermediate was purified by chromatography (silica gel, CHCl₃/MeOH/H₂O 40:55:5) yielding approximately 100mg of material. Hydrogenation of this intermediate in aqueous ethanol, employing 5%Pd/C yielded the title compound. LC/MS and NMR data are available.

### Example 2

### Sleep indication in mice

The method which detects sedative activity following the protocol described by Simon et al. (*J*. *Pharmacol.* Paris, 13:241-252, 1982).

Mice (10 per group) are placed in Plexiglass cages (20 x 10 x 10 cm) and administered the test substance, propofol, produced as above as an i.v. bolus in two seconds. The latency to sleep and the occurrence of sedation/sleep are noted over a period of one hour. Sleep is indicated by the loss of the righting reflex. Animals within a group are tested sequentially and the test is performed blind. The test substance will be evaluated in 5 escalating doses. Unmodified propoful (16 mg per kg) administered in the same experimental conditions, will be used as a reference compound. The LD₅₀ for hypnotic activity is calculated following the method of Lichtfield and Wilcoxin (*J. Pharmacol. Exp. Ther.* 96:99-113, 1949).

### Lethal dose 50 (LD₅₀) in mice

The method, which determines the acute dose of a test substance causing 50% of death in a given animal species, follows the method described by Lichtfield and Wilcoxin.

After an 18 hour period of food deprivation but free access to water, mice will be administered the test substance as an i.v. bolus in two seconds. The appearance of morbidity, including local reaction and mortality, are noted for a period of 7 days, during which the animals have free access to food and water.

Ten mice are studied per group. The test is performed blind.

The test substance will be evaluated at 5 escalating doses.

No reference substance and no control group are offered for this experiment.

The LD₅₀ is calculated at the end of the testing following the method of Lichtfield and Wilcoxin see *J. Pharmacol. Exp. Ther.* 96:99-113, 1949 above.

Variations of the present invention will suggest themselves to those skilled in the art, and are within the scope of the following claims:

## Claims

1. A compound having the general formula I: 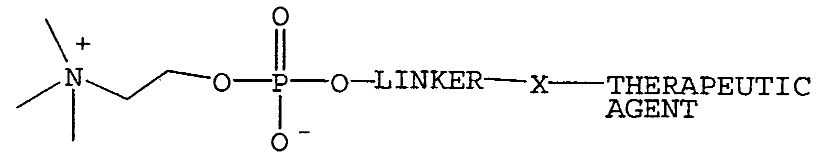 wherein the LINKER is one or more of the groups selected from the group consisting of (i) substituted or unsubstituted alkyl, (ii) substituted or unsubstituted alkenyl, (iii) substituted or unsubstituted alkanoyl, (iv) substituted or unsubstituted alkenoyl wherein the double bond is cis, and (v)(ortho or para) carbonyl-substituted aryl; and
wherein the subtituent is each an independent group or linked together thereby forming a ring; and
wherein X is one or more substituted or unsubstituted group containing one or more O, N, or S atom and
wherein the substituent is each an independent group or linked together thereby forming a ring; and
wherein the therapeutic agent is selected from the group consisting of alcohol- containing water-insoluble steroids and another alcohol containing compounds.

2. A compound according to claim 1, wherein (i) said alkyl has the formula CR₁R₂, (ii) said alkenyl has the formula CR₁=CR₃-CR₄, (iii) said alkanoyl has the formula CR₁R₂-CR₃R₄-CR₅R₆-CO, (iv) said alkenoyl has the formula CR₁R₂-CR₃=CR₄-CO and wherein the double bond is cis, and (v) said substituted aryl has the formula aryl-CR₁R₂; and
wherein R₁, R₂, R₃, R₄, R₅, and R₆ are the same or different and are selected from the group consisting of
(i) hydrogen;
(ii) linear, branched, and unsaturated C₁₋₁₂-alkyl;
(iii) substituted C₁₋₈-alkyl, wherein the substituent is selected from the group consisting of Y1-Y24, wherein
Y1 is hydroxy,
Y2 is C₁₋₈-alkoxy,
Y3 is carbo-C₁₋₈-alkoxy,
Y4 is C₁₋₈-alkylamino,
Y5 is di-C₁₋₈-alkylamino,
Y6 is C₆₋₁₂-arylamino,
Y7 is C₆₋₁₂- aryloxy,
Y8 is amino,
Y9 is amino-C₂-C₈-alkoxy,
Y10 is C₁₋₈-alkylthio,
Y11 is C₆₋₁₂-arylthio,
Y12 is acetamido,
Y13 is mercapto,
Y14 is benzamido,
Y15 is carboxamido,
Y16 is phthalimido,
Y17 is guanidino,
Y18 is ureido,
Y19 is isothioureido,
Y20 is carboxy,
Y21 is (C₆₋₁₂) aryl- (C₁₋₈) alkyl,
Y22 is (C₆₋₁₂) aryl- (C₂₋₈) alkenyl,
Y23 is aromatic heterocyclo (C₁₋₈) alkyl,
and Y24 is aromatic heterocyclo (C₂₋₈) alkenyl wherein the heterocyclic group of Y23 and Y24 have 5 - 10 ring atoms and comprises up to two O, N, or S heteroatoms; and
(iv) substituted Y21 or substituted Y23 wherein the substituent is selected from the group consisting of Y1, Y2, Y4, Y5, Y7, Y8, Y12, Y14, Y17-Y20, and Y25-Y29 wherein
Y25 is halogen,
Y26 is C₁₋₈-alkyl,
Y27 is amino-C₁₋₈-alkyl,
Y28 is C₆₋₁₂-aroyl, and
Y29 is C₁₋₈-alkanoyl.

3. A compound according to claim 2, wherein said R₁ and R₂; R₁ and R₃; R₂ and R₃; R₃ and R₄; R₃ and R₅; and R₅ and R₆ are linked together thereby forming:
(i) a ring of three to six carbon atoms, or
(ii) a ring of two to five carbon atoms and one O, or S heteroatom, or substituted heteroatom NR₇; wherein R₇ is selected from the group consisting of Y21, Y26, Y28, Y29, and Y30-Y31, wherein
Y30 is C₃₋₈-alkenyl, and
Y31 is C₆₋₁₂-aryl.

4. A compound according to claim 2 wherein the group containing one or more O, N, or S atom is selected from the group consisting of O, (O) CO, NR₈, NR₈ CO, NR₈ CO NR₉, NR₈ (SO₂), NR₈ CS, NR₈ CS NR₉, ONR₈, ONR₈CO, NR₈ (O), NR₈ (O) CO, nitrogen heterocycles, amide and urea internal in therapeutic agent; and
wherein R₈ and R₉ are the same or different and are selected from the group consisting of
(i) hydrogen;
(ii) linear, branched, and unsaturated C₁₋₁₂-alkyl;
(iii) substituted C₁₋₈-alkyl, wherein the substituent is selected from the group consisting of Y1-Y13 and Y15-Y25;
(iv) substituted Y21 or substituted Y23 wherein the substituent is selected from the group consisting of Y1, Y2, Y4, Y5, Y7, Y8, Y12, Y14, Y17-Y20, and Y25-Y29.

5. A compound according to claim 4 wherein R₈ and R₉ are linked together thereby forming
(i) a ring of three to six carbon atoms, or
(ii) a ring of two to five carbon atoms and one O, or S heteroatom, or substituted heteroatom NR₇; wherein R₇ is selected from the group consisting of Y21, Y26, and Y28-Y31.

6. A compound according to claim 4 wherein R₈, R₉, or both are connected to the therapeutic agent molecule thereby forming alkylene bridge of from one to five carbon atoms and one or two O, S or NR₇ heteroatoms; wherein R₇ is selected from the group consisting of Y21, Y26, Y28-Y31, and the pharmaceutically acceptable salts thereof.

7. A compound according to claim 5 wherein R₈, R₉, or both are connected to the therapeutic agent molecule thereby forming alkylene bridge of from one to five carbon atoms and one or two O, S or NR₇ heteroatoms; wherein R₇ is selected from the group consisting of Y21, Y26, Y28-Y31; and the pharmaceutically acceptable salts thereof.

8. A compound according to claim 2, wherein said (*ortho* or *para*) carbonyl-substituted aryl is selected from the group consisting of *ortho*-CR₁R₂-substituted aryl-CO, substituted aryl-*ortho*-CR₃R₄-CO, substituted aryl-*ortho*- CR₃R₄-CR₅R₆-CO, substituted aryl-*ortho*-CR₃=CR₄-CO wherein the double bond is *cis, ortho*-CR₁R₂-substituted aryl-CR₅R₆-CO, and substituted aryl-(*ortho* or *para*)-CO.

9. A compound according to claim 2, wherein said aryl is selected from the group consisting of benzene, naphthalene, pyridine, pyrrole, thiophene, furan, imidazole, thiazole, oxazole, pyrimidine, indole, benzimidazole, benzthiazole, benzofuran, benzothiophene and quinoline, each bearing one or more of the group consisting of hydrogen, C₁₋₈-alkyl, C₁₋₈-alkoxy, F, Cl, Br, C₁₋₈-alkoxycarbonyl, amino, substituted amino, nitro, C₁₋₈-alkylthio, C₁₋₈-alkylsulfoxido, and C₁₋₈-alkylsulfono.

10. A compound according to claim 2, wherein R₁ is hydrogen.

11. A compound according to claim 2, wherein R₁ and R₂ are hydrogen.

12. A compound according to claim 1, wherein the therapeutic agent is selected from the group cohsisting of Propofol and related anesthetic or sedative compounds.

13. A compound according to claim 1, wherein said water-insoluble steroids are selected from the group consisting of (i) testosterone, (ii) cardiotonic steroids selected from the group consisting of digitoxigenin, digoxigenin and ouabugenin, (iii) dehydroepiandrosterone (DHEA), (iv) etiocholanolone, (v) pregnenolone, (vi) estradiol, (vii) estrone, (viii) dexamethasone and (ix) hydrocortisone.

14. A compound according to claim 1, further comprises one or more of the ingredients selected from the group consisting of pharmaceutically-acceptable carriers, diluents, fillers, salts, buffers, preservatives, antioxidants, a binder, an excipient, a disintegrating agent, a lubricant, and a sweetening agent.

15. A compound according to claim 1 incorporated into tablets, capsules or elixirs for oral administration; suppositories for rectal administration; sterile solutions or suspensions for injectable administration; sterile solutions for ocular or internasal administration.

16. A compound having the general formula I: 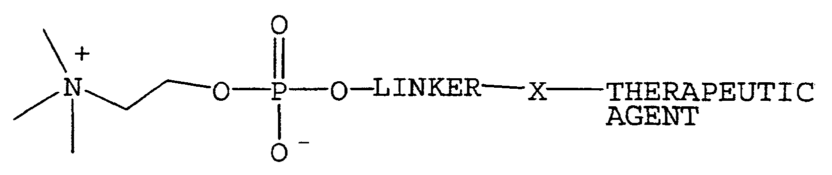 wherein the LINKER is a substituted alkenyl of formula CR₁R₂-CR₃=CR₄-CO, wherein R₁, R₃, and R₄ are hydrogen and wherein the double bond is *trans*, and
wherein X is O and
wherein the therapeutic agent is 2',6'-diisopropyl phenol.

17. A compound having the general formula I: 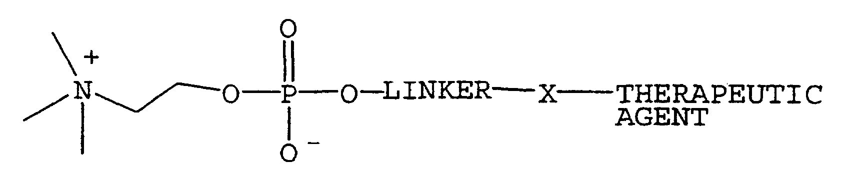 wherein the LINKER is a substituted alkanoyl of formula CR₁R₂-CR₃R₄-CR₅R₆-CO, wherein R₁, R₂, R₃, R₄, R₅, and R₆ are H, and
wherein X is O and
wherein the therapeutic agent is 2',6'-diisoproyl phenol.

18. A method for enabling potential therapeutic agents to be rendered soluble comprising the steps of inserting one or more linker moieties having one or more primary alcohol group between a phosphocholine or a phosphocholine congener to the therapeutic agents having one or more alcohol group.

19. A method for increasing the bioavailability of pharmaceutical agents on administration to a mammal comprising the steps of derivatizing the agent with one or more linker moieties, producing an intermediate, recovering and coupling the intermediate with phosphocholine or a phosphocholine-congener to the linkers and producing a final derivative, wherein the agent in derivative form is significantly more soluble in aqueous media than the agent in non-derivatized form.

20. The method of claim 19 wherein the pharmaceutical agent is propofol.

21. A pharmaceutical formulation for treating a mammal suffering from cancer comprising an isolated phosphocholine linked via a linker to paclitaxel and a physiologically acceptable vehicle, carrier, binder, preservative, stabilizer, or flavor as called for by accepted pharmaceutical practice, wherein the linker is one or more of the groups selected from the group consisting of (i) substituted or unsubstituted alkyl, (ii) substituted or unsubstituted alkenyl, (iii) substituted or unsubstituted alkanoyl, (iv) substituted or unsubstituted alkenoyl wherein the double bond is cis, and (v) (ortho or para) carbonyl-substituted aryl; and wherein the substituent is each an independent group of linked together thereby forming a ring.

## Patentansprüche

1. Verbindung mit der allgemeinen Formel I: 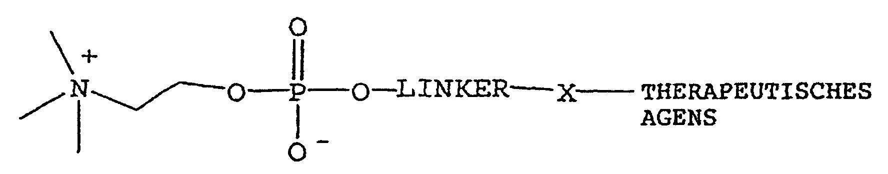 worin der LINKER eine oder mehrere der Gruppen, ausgewählt aus der Gruppe bestehend aus (i) substituiertem oder unsubstituiertem Alkyl, (ii) substituiertem oder unsubstituiertem Alkenyl, (iii) substituiertem oder unsubstituiertem Alkanoyl, (iv) substituiertem oder unsubstituiertem Alkenoyl, worin die Doppelbindung cis ist, und (v) (*ortho* oder *para*) Carbonyl-substituiertem Aryl, ist; und
worin der Substituent jeweils eine unabhängige Gruppe ist oder zusammengebunden ist und dadurch einen Ring bildet; und
worin X eine oder mehrere substituierte oder unsubstituierte Gruppe(n) bedeutet, die ein oder mehrere O-, N-, oder S-Atom(e) enthält bzw. enthalten; und
worin der Substituent jeweils eine unabhängige Gruppe ist oder zusammengebunden ist und dadurch einen Ring bildet; und
worin das therapeutische Agens ausgewählt wird aus der Gruppe bestehend aus Alkohol enthaltenden wasserunlöslichen Steroiden und anderen Alkohol enthaltenden Verbindungen.

2. Verbindung nach Anspruch 1, worin (i) besagtes Alkyl die Formel CR₁R₂ hat, (ii) besagtes Alkenyl die Formel CR₁=CR₃-CR₄ hat, (iii) besagtes Alkanoyl die Formel CR₁R₂-CR₃R₄-CR₅R₆-CO hat, (iv) besagtes Alkenoyl die Formel CR₁R₂-CR₃=CR₄-CO hat und worin die Doppelbindung cis ist, und (v) besagtes substituiertes Aryl die Formel Aryl-CR₁R₂ hat; und
worin R₁, R₂, R₃, R₄, R₅ und R₆ gleich oder unterschiedlich sind und ausgewählt werden aus der Gruppe bestehend aus:
(i) Wasserstoff;
(ii) linearem, verzweigtem und ungesättigtem C₁₋₁₂-Alkyl;
(iii) substituiertem C₁₋₈-Alkyl, worin der Substituent ausgewählt wird aus der Gruppe bestehend aus Y1-Y24, worin
Y1 Hydroxy ist,
Y2 C₁₋₈-Alkoxy ist,
Y3 Carbo-C₁₋₈-alkoxy ist,
Y4 C₁₋₈-Alkylamino ist,
Y5 Di-C₁₋₈-alkylamino ist,
Y6 C₆₋₁₂-Arylamino ist,
Y7 C₆₋₁₂-Aryloxy ist,
Y8 Amino ist,
Y9 Amino-C₂-C₈-alkoxy ist,
Y10 C₁₋₈-Alkylthio ist,
Y11 C₆₋₁₂-Arylthio ist,
Y12 Acetamido ist,
Y13 Mercapto ist,
Y14 Benzamido ist,
Y15 Carboxamido ist,
Y16 Phthalimido ist,
Y17 Guanidino ist,
Y18 Ureido ist,
Y19 Isothioureido ist,
Y20 Carboxy ist,
Y21 (C₆₋₁₂)Aryl-(C₁₋₈)alkyl ist,
Y22 (C₆₋₁₂)Aryl-(C₂₋₈)alkenyl ist,
Y23 aromatisches Hetercyclo(C₁₋₈)alkyl ist, und
Y24 aromatisches Hetercyclo(C₂₋₈)alkenyl ist, worin die heterocyclischen Gruppen von Y23 und Y24 5-10 Ringatome haben und bis zu zwei O-, N-oder S-Heteroatome umfassen; und
(iv) substituiertem Y21 oder substituiertem Y23, worin der Substituent ausgewählt wird aus der Gruppe bestehend aus Y1, Y2, Y4, Y5, Y7, Y8, Y12, Y14, Y17-Y20 und Y25-Y29, worin
Y25 Halogen ist,
Y26 C₁₋₈-Alkyl ist,
Y27 Amino-C₁₋₈-alkyl ist,
Y28 C₆₋₁₂-Aroyl ist und
Y29 C₁₋₈-Alkanoyl ist.

3. Verbindung nach Anspruch 2, worin besagte R₁ und R₂; R₁ und R₃; R₂ und R₃; R₃ und R₄; R₃ und R₅; und R₅ und R₆ zusammengebunden sind und dadurch
(i) einen Ring mit drei bis sechs Kohlenstoff-Atomen oder
(ii) einen Ring mit zwei bis fünf Kohlenstoff-Atomen und einem O- oder S-Heteroatom oder substituierten Heteroatom NR₇ bilden, worin R₇ ausgewählt wird aus der Gruppe bestehend aus Y21, Y26, Y28, Y29 und Y30-Y31, worin
Y30 C₃₋₈-Alkenyl ist und
Y31 C₆₋₁₂-Aryl ist.

4. Verbindung nach Anspruch 2, worin die Gruppe, welche ein oder mehrere O-, N-oder S-Atom(e) enthält; ausgewählt wird aus der Gruppe bestehend aus O, (O)CO, NR₈, NR₈ CO, NR₈ CO NR₉, NR₈(SO₂), NR₈ CS, NR₈ CS NR₉, ONR₈, ONR₈CO, NR₈(O), NR₈(O)CO, Stickstoff-Heterocyclen, Amid und Harnstoff innerhalb des therapeutischen Agens, und
worin R₈ und R₉ gleich oder unterschiedlich sind und ausgewählt werden aus der Gruppe bestehend aus:
(i) Wasserstoff;
(ii) linearem, verzweigtem und ungesättigtem C₁₋₁₂-Alkyl;
(iii) substituiertem C₁₋₈-Alkyl, worin der Substituent ausgewählt wird aus der Gruppe bestehend aus Y1-Y13 und Y15-Y25;
(iv) substituiertem Y21 oder substituiertem Y23, worin der Substituent ausgewählt wird aus der Gruppe bestehend aus Y1, Y2, Y4, Y5, Y7, Y8, Y12, Y14, Y17-Y20 und Y25-Y29.

5. Verbindung nach Anspruch 4, worin R₈ und R₉ zusammengebunden sind und dadurch
(i) einen Ring mit drei bis sechs Kohlenstoff-Atomen oder
(ii) einen Ring mit zwei bis fünf Kohlenstoff-Atomen und einem O- oder S-Heteroatom oder substituierten Heteroatom NR₇ bilden, worin R₇ ausgewählt wird aus der Gruppe bestehend aus Y21, Y26 und Y28-Y31.

6. Verbindung nach Anspruch 4, worin R₈, R₉ oder beide mit dem Molekül des therapeutischen Agens verbunden sind und dadurch eine Alkylen-Brücke mit einem bis fünf Kohlenstoff-Atomen und einem oder zwei O-, S- oder NR₇-Heteroatomen bilden; worin R₇ ausgewählt wird aus der Gruppe bestehend aus Y21, Y26, Y28-Y31, und die pharmazeutisch akzeptablen Salze davon.

7. Verbindung nach Anspruch 5, worin R₈, R₉ oder beide mit dem Molekül des therapeutischen Agens verbunden sind und dadurch eine Alkylen-Brücke mit einem bis fünf Kohlenstoff-Atomen und einem oder zwei O-, S- oder NR₇-Heteroatomen bilden, worin R₇ ausgewählt wird aus der Gruppe bestehend aus Y21, Y26, Y28-Y31, und die pharmazeutisch akzeptablen Salze davon.

8. Verbindung nach Anspruch 2, worin besagtes (*ortho* oder *para*) Carbonylsubstituiertes Aryl ausgewählt wird aus der Gruppe bestehend aus *ortho*-CR₁R₂substituiertem Aryl-CO, substituiertem Aryl-*ortho*-CR₃R₄-CO, substituiertem Aryl*ortho-* CR₃R₄-CR₅R₆-CO, substituiertem Aryl-*ortho*-CR₃=CR₄-CO, worin die Doppelbindung *cis* ist, *ortho*-CR₁R₂-substituiertem Aryl-CR₅R₆-CO und substituiertem Aryl- (*ortho* oder *para*) -CO.

9. Verbindung nach Anspruch 2, worin besagtes Aryl ausgewählt wird aus der Gruppe bestehend aus Benzol, Naphthalin, Pyridin, Pyrrol, Thiophen, Furan, Imidazol, Thiazol, Oxazol, Pyrimidin, Indol, Benzimidazol, Benzthiazol, Benzofuran, Benzothiophen und Chinolin, wobei jedes eines oder mehrere aus der Gruppe bestehend aus Wasserstoff, C₁₋₈-Alkyl, C₁₋₈-Alkoxy, F, Cl, Br, C₁₋₈-Alkoxycarbonyl, Amino, substituiertem Amino, Nitro, C₁₋₈-Alkylthio, C₁₋₈-Alkylsulfoxido und C₁₋₈-Alkylsulfono trägt.

10. Verbindung nach Anspruch 2, worin R₁ Wasserstoff ist.

11. Verbindung nach Anspruch 2, worin R₁ und R₂ Wasserstoff sind.

12. Verbindung nach Anspruch 1, worin das therapeutische Agens ausgewählt wird aus der Gruppe bestehend aus Propofol und verwandten anästhetischen oder sedativen Verbindungen.

13. Verbindung nach Anspruch 1, worin besagte wasserunlösliche Steroide ausgewählt werden aus der Gruppe bestehend aus (i) Testosteron, (ii) herzstärkenden Steroiden ausgewählt aus der Gruppe bestehend aus Digitoxigenin, Digoxigenin und Ouabagenin, (iii) Dehydroepiandrosteron (DHEA), (iv) Etiocholanolon, (v) Pregnenolon, (vi) Östradiol, (vii) Östron, (viii) Dexamethason und (ix) Hydrocortison.

14. Verbindung nach Anspruch 1, welche weiter einen oder mehrere der Bestandteile umfasst, die ausgewählt werden aus der Gruppe bestehend aus pharmazeutisch akzeptablen Trägern, Verdünnungsmitteln, Füllstoffen, Salzen, Puffern, Konservierungsmitteln, Antioxidantien, einem Bindemittel, einem Arzneistoffträger, einem Zersetzungsmittel, einem Gleitmittel und einem Süßstoff.

15. Verbindung nach Anspruch 1, die in Tabletten, Kapseln oder Elixieren für eine orale Verabreichung; in Suppositorien für eine rektale Verabreichung; in sterilen Lösungen oder Suspensionen für eine injizierbare Verabreichung und in sterilen Lösungen für eine Verabreichung am Auge oder eine intranasale Verabreichung enthalten ist.

16. Verbindung mit der allgemeinen Formel I: 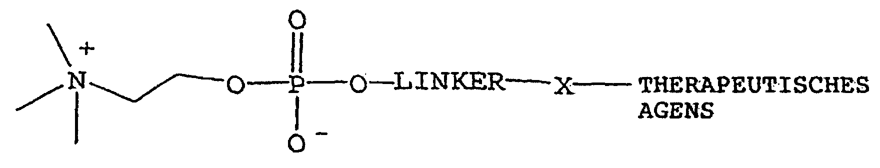 worin der LINKER ein substituiertes Alkenyl mit der Formel CR₁R₂-CR₃=CR₄-CO ist, worin R₁, R₃ und R₄ Wasserstoff sind und worin die Doppelbindung *trans* ist, und
worin X O ist und
worin das therapeutische Agens 2',6'-Diisopropylphenol ist.

17. Verbindung mit der allgemeinen Formel I: 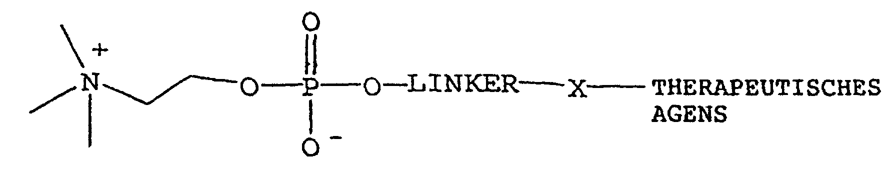 worin der LINKER ein substituiertes Alkanoyl mit der Formel CR₁R₂-CR₃R₄-CR₅R₆-CO ist, worin R₁, R₂, R₃, R₄, R₅ und R₆ H sind, und
worin X O ist und
worin das therapeutische Agens 2',6'-Diisopropylphenol ist.

18. Verfahren zum Ermöglichen, dass potenzielle therapeutische Agentien löslich gemacht werden, umfassend die Schritte des Inserierens einer oder mehrerer Linker-Komponenten, die eine oder mehrere primäre Alkohol-Gruppen haben, zwischen ein Phosphocholin oder einen Verwandten des Phosphocholins und die therapeutischen Agentien, die eine oder mehrere Alkohol-Gruppen haben.

19. Verfahren zum Erhöhen der Bioverfügbarkeit pharmazeutischer Agentien nach Verabreichung an einen Säuger, umfassend die Schritte der Derivatisierung des Agens mit einer oder mehreren Linker-Komponenten, der Herstellung eines Zwischenprodukts, der Wiedergewinnung und Kopplung des Zwischenprodukts mit Phosphocholin oder einem Verwandten des Phosphocholins an die Linker und der Herstellung eines endgültigen Derivats, worin das Agens in seiner Derivat-Form in wässrigen Medien signifikant besser löslich ist als das Agens in nicht-derivatisierter Form.

20. Verfahren nach Anspruch 19, worin das pharmazeutische Agens Propofol ist.

21. Pharmazeutische Formulierung zum Behandeln eines Säugers, der an Krebs leidet, umfassend ein isoliertes Phosphocholin, das über einen Linker an Paclitaxel gebunden ist, und ein physiologisch akzeptables Vehikel, Träger, Bindemittel, Konservierungsmittel, Stabilisator oder Geschmacksstoff, wie es die anerkannte pharmazeutische Praxis verlangt, worin der Linker eine oder mehrere der Gruppen, ausgewählt aus der Gruppe bestehend aus (i) substituiertem oder unsubstituiertem Alkyl, (ii) substituiertem oder unsubstituiertem Alkenyl, (iii) substituiertem oder unsubstituiertem Alkanoyl, (iv) substituiertem oder unsubstituiertem Alkenoyl, worin die Doppelbindung cis ist, und (v) (*ortho* oder *para*) Carbonyl-substituiertem Aryl, ist; und worin der Substituent jeweils eine unabhängige Gruppe ist oder zusammengebunden ist und dadurch einen Ring bildet.

## Revendications

1. Composé ayant la formule générale I : 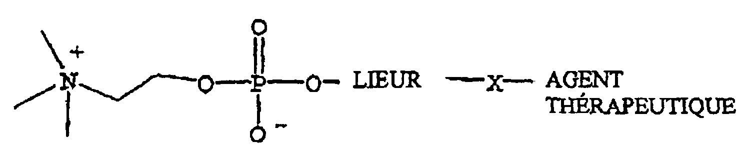 dans laquelle le LIEUR représente un ou plusieurs des groupes choisis dans le groupe constitué par (i) un alkyle substitué ou non substitué, (ii) un alcényle substitué ou non substitué, (iii) un alcanoyle substitué ou non substitué, (iv) un alcénoyle substitué ou non substitué dans lequel la double liaison est cis, et (v) un aryle substitué par un carbonyle (en *ortho ou para*) ; et
dans laquelle le substituant est chacun un groupe indépendant ou lié ensemble pour former ainsi un cycle ; et
dans laquelle X représente un ou plusieurs groupes substitués ou non substitués contenant un ou plusieurs atomes de O, N ou S ; et
dans laquelle le substituant est chacun un groupe indépendant ou lié ensemble pour former ainsi un cycle ; et
dans laquelle l'agent thérapeutique est choisi dans le groupe constitué de stéroïdes insolubles dans l'eau contenant un alcool et de composés contenant un autre alcool.

2. Composé selon la revendication 1, dans lequel (i) ledit alkyle a la formule CR₁R₂, (ii) ledit alcényle a la formule CR₁=CR₃-CR₄, (iii) ledit alcanoyle a la formule CR₁R₂-CR₃R₄-CR₅R₆-CO, (iv) ledit alcénoyle a la formule CR₁R₂-CR₃=CR₄-CO et dans lequel la double liaison est cis, et (v) ledit aryle substitué a la formule aryl-CR₁R₂ ; et
dans lequel R₁, R₂, R₃, R₄, R₅, et R₆ sont identiques ou différents et sont choisis dans le groupe constitué par :
(i) un hydrogène ;
(ii) un alkyle en C₁₋₁₂ linéaire, ramifié, et insaturé ;
(iii) un alkyle en C₁₋₈ substitué, dans lequel le substituant est choisi dans le groupe constitué de Y1 -Y24, dans lequel :
Y1 est un hydroxy,
Y2 est un alcoxy en C₁₋₈,
Y3 est un carbo-alcoxy en C₁₋₈,
Y4 est un alkylamino en C₁₋₈,
Y5 est un di-alkylamino en C₁₋₈,
Y6 est un arylamino en C₆₋₁₂,
Y7 est un aryloxy en C₆₋₁₂,
Y8 est un amino,
Y9 est un amino-alcoxy en C₂₋₈,
Y10 est un alkylthio en C₁₋₈,
Y11 est un arylthio en C₆₋₁₂,
Y12 est un acétamido,
Y13 est un mercapto,
Y14 est un benzamido,
Y15 est un carboxamido,
Y16 est un phtalimido,
Y17 est un guanidino,
Y18 est un uréido,
Y19 est un isothlouréido,
Y20 est un carboxy,
Y21 est un aryle en (C₆₋₁₂)-alkyle en (C₁₋₈),
Y22 est un aryle en (C₆₋₁₂)-alcényle en (C₂₋₈),
Y23 est un hétérocyclo-alkyle en (C₁₋₈) aromatique,
et Y24 est un hétérocyclo-alcényle en (C₂₋₈) aromatique où le groupe hétérocyclique de Y23 et Y24 a de 5 à 10 atomes de cycle et comprend jusqu'à deux hétéroatomes de O, N ou S ; et
(iv) Y21 substitué ou Y23 substitué dans lequel le substituant est choisi dans le groupe constitué de Y1, Y2, Y4, Y5, Y7, Y8, Y12, Y14, Y17-Y20 et Y25-Y29 dans lequel :
Y25 est un halogène,
Y26 est un alkyle en C₁₋₈,
Y27 est un amino-alkyle en C₁₋₈,
Y28 est un aroyle en C₆₋₁₂, et
Y29 est un alcanoyle en C₁₋₈.

3. Composé selon la revendication 2, dans lequel lesdits R₁ et R₂ ; R₁ et R₃ ; R₂ et R₃ ; R₃ et R₄ ; R₃ et R₅ ; et R₅ et R₆ sont liés ensemble pour former ainsi :
(i) un cycle de trois à six atomes de carbone, ou
(ii) un cycle de deux à cinq atomes de carbone et un hétéroatome de O ou S, ou un hétéroatome de NR₇ substitué ; dans lequel R₇ est choisi dans le groupe constitué de Y21, Y26, Y28, Y29, et Y30-Y31, dans lequel :
Y30 est un alcényle en C₃₋₈, et
Y31 est un aryle en C₆₋₁₂.

4. Composé selon la revendication 2, dans lequel le groupe contenant un ou plusieurs atomes de O, N ou S sont choisis dans le groupe constitué de O, (O) CO, NR₉, NR₈, NR₈ CO, NR₈ CO NR₉, NR₈ (SO₂), NR₈ CS, NR₈ CS NR₉, ONR₈, ONR₈CO, NR₈ (O), NR₈ (O) CO, d'hétérocycles azotés, d'amide et d'urée interne dans l'agent thérapeutique ; et
dans lequel R₈ et R₉ sont identiques ou différents et sont choisis dans le groupe constitué par :
(i) un hydrogéne ;
(ii) un alkyle en C₁₋₁₂ linéaire, ramifié, et insaturé;
(iii) un alkyle en C₁₋₈ substitué, dans lequel le substituant est choisi dans le groupe constitué de Y1-Y13 et Y15-Y25 ;
(iv) Y21 substitué ou Y23 substitué dans lequel le substitué est choisi dans le groupe constitué de Y1, Y2, Y4, Y5, Y7, Y8, Y12, Y14, Y17-Y20, et Y25-Y29.

5. Composé selon la revendication 4, dans lequel R₈ et R₉ sont liés ensemble pour former ainsi :
(i) un cycle de trois à six atomes de carbone, ou
(ii) un cycle de deux à cinq atomes de carbone et un hétéroatome de O ou S, ou un hétéroatome de NR₇ substitué ; dans lequel R₇ est choisi dans le groupe constitué de Y21, Y26, et Y28-Y31.

6. Composé selon la revendication 4, dans lequel R₈, R₉, ou les deux sont reliés à la molécule de l'agent thérapeutique pour former ainsi un pont alkylène ayant d'un à cinq atomes de carbone et un ou deux hétéroatomes de O, S ou NR₇ ; dans lequel R₇ est choisi dans le groupe constitué de Y21, Y26, Y28-Y31, et les sels pbarmaceutiquement acceptables de ceux-ci.

7. Composé selon la revendication 5, dans lequel R₈, R₉, ou les deux sont reliés à la molécule de l'agent thérapeutique pour former ainsi un pont alkylène ayant d'un à cinq atomes de carbone et un ou deux hétéroatomes de O, S ou NR₇ ; dans lequel R₇ est choisi dans le groupe constitué de Y21, Y26, Y28-Y31, et les sels pharmaceutiquement acceptables de ceux-ci.

8. Composé selon la revendication 2, dans lequel ledit aryle substitué par un carbonyle (*ortho* ou *para*) est choisi dans le groupe constitué par un *ortho*-CR₁R₂-aryle substitué CO, un aryle substitué-*ortho*-CR₃R₄-CO, un aryle substitué-*ortho*-CR₃R₄-CR₅R₆-CO, un aryle substitué-*ortho*-CR₃=CR₄-CO dans lequel la double liaison est cis, un *ortho*-CR₁R₂-aryle substitué-CR₅R₆-CO, et un aryle substitué (*ortho* ou *para*)-CO.

9. Composé selon la revendication 2, dans lequel ledit aryle est choisi dans le groupe constitué de benzène, naphtalène, pyridine, pyrrole, thiofène, furanne, imidazole, thiazole, oxazole, pyrimidine, indole, benzimidazole, benzothiazole, benzofuranne, benzothiofène et quinoléine, portant chacun un ou plusieurs membres du groupe constitué d'un hydrogène, un alkyle en C₁₋₈, un alcoxy en C₁₋₈, F, Cl, Br, un alcoxycarbonyle en C₁₋₈, un amino, un amino substitué, un nitro, un alkylthio en C₁₋₈, un alkylsulfoxydo en C₁₋₈, et un alkylsulfono en C₁₋₈.

10. Composé selon la revendication 2, dans lequel R₁ est un hydrogène.

11. Composé selon la revendication 2, dans lequel R₁ et R₂ sont tous deux un hydrogène.

12. Composé selon la revendication 1, dans lequel l'agent thérapeutique est choisi dans le groupe constitué de propofol et de composés anesthésiques ou sédatifs associés.

13. Composé selon la revendication 1, dans lequel lesdits stéroïdes insolubles dans l'eau sont choisis dans le groupe constitué par (i) la testostérone, (ii) des stéroïdes cardiotoniques choisis dans le groupe constitué de digitoxigénine, digoxigénine et ouabugénine, (iii) la déhydroépiandrostérone (DHEA), (iv) l'étiocholanolone, (v) la prégnènolone, (vi) l'estradiol, (vii) l'oestrone, (viii) la dexaméthasone et (ix) l'hydrocortisone.

14. Composé selon la revendication 1, qui comprend en outre un ou plusieurs des ingrédients choisis dans le groupe constitué par des véhicules, diluants, matières de charge, sels, tampons, conservateurs, antioxydants, un liant, un excipient, un délitant, un lubrifiant, et un agent édulcorant pharmaceutiquement acceptables.

15. Composé selon la revendication 1, incorporé dans des comprimés, capsules ou élixirs pour une administration par voie orale ; des suppositoires pour une administration par voie rectale ; des solutions ou suspensions stériles pour une administration de préparation injectable ; des solutions stériles pour une administration par voie oculaire ou intranasale.

16. Composé ayant la formule générale 1 : 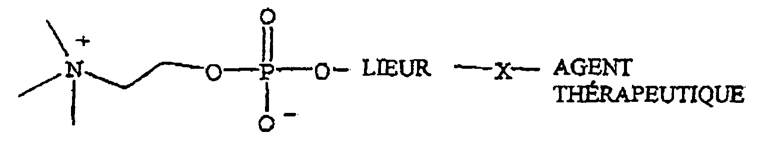 dans laquelle le LIEUR est un alcényle substitué de formule CR₁R₂-CR₃=CR₄-CO, dans laquelle R₁, R₃, et R₄ sont un hydrogène et dans laquelle la double liaison est trans, et
dans laquelle X est O et
dans laquelle l'agent thérapeutique est le 2',6'-diisopropylphénol.

17. Composé ayant la formule générale I : 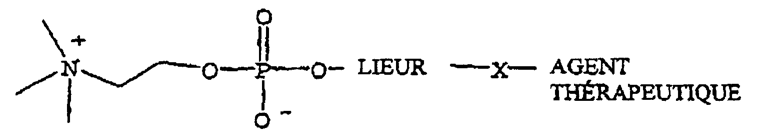 dans laquelle le LIEUR est un alcanoyle substitué de formule CR₁R₂-CR₃R₄-CR₅R₆-CO, dans laquelle R₁, R₂, R₃, R₄, R₅, et R₆ sont H, et
dans laquelle X est O et
dans laquelle l'agent thérapeutique est le 2',6'-diisopropylphénol.

18. Procédé permettant à des agents thérapeutiques potentiels d'être rendus solubles comprenant les étapes d'insertion d'un ou plusieurs fragments de lieur ayant un ou plusieurs premiers groupes alcool entre un congénère de phosphocholine ou un phosphocholine aux agents thérapeutiques ayant un ou plusieurs groupes alcool.

19. Procédé d'accroissement de la biodisponibilité d'agents pharmaceutiques des l'administration à un mammifère comprenant les étapes de dérivation de l'agent avec un ou plusieurs fragments de lieur, production d'un intermédiaire, récupération et couplage de l'intermédiaire avec la phosphocholine ou un congénère de phosphocholine aux lieurs et production d'un dérivé final, dans lequel l'agent sous une forme dérivée est significativement plus soluble dans un milieu aqueux que l'agent sous une forme non dérivée.

20. Procédé selon la revendication 19, dans lequel l'agent pharmaceutique est le propofol.

21. Préparation pharmaceutique pour traiter un mammifère souffrant d'un cancer comprenant une phosphocholine isolée liée via un lieur au paclitaxel et un véhicule, un support, un liant, un conservateur, un stabilisant physiologiquement acceptables, ou un arôme tel que confirmé dans la pratique pharmaceutique acceptée, dans laquelle le lieur est un ou plusieurs des groupes choisis dans le groupe constitué par (i) un alkyle substitué ou non substitué, (ii) un alcényle substitué ou non substitué, (iii) un alcanoyle substitué ou non substitué, (iv) un alcénoyle substitué ou non substitué dans lequel la double liaison est cis, et (v) un aryle substitué (en *ortho* ou *para*) par un carbonyle; et dans lequel le substituant est chacun un groupe indépendant ou lié ensemble pour former ainsi un cycle.
